**Europäisches Patentamt**

**European Patent Office**

**Office européen des brevets**

(11) Publication number: **0 022 655**
**B1**

(12) **EUROPEAN PATENT SPECIFICATION**

(45) Date of publication of patent specification: **18.09.85**

(21) Application number: **80302318.3**

(22) Date of filing: **09.07.80**

(51) Int. Cl.⁴: **A 61 C 13/083**

(54) Dental constructs and tools and production thereof.

(30) Priority: **13.07.79 US 57399**

(43) Date of publication of application:
**21.01.81 Bulletin 81/03**

(45) Publication of the grant of the patent:
**18.09.85 Bulletin 85/38**

(84) Designated Contracting States:
**AT BE CH DE FR GB IT LI LU NL SE**

(56) References cited:
**DD-A- 132 332**
**DE-A-2 711 219**
**DE-B-2 347 591**
**GB-A-1 441 082**
**US-A-3 689 293**
**US-A-3 732 087**

**DENTAL-LABOR, Vol. XXIII, No. 9, 1975,**
**"Entwicklung von neuem künstlichem**
**Zahnmaterial", page 977**

(73) Proprietor: **Corning Glass Works**
**Houghton Park**
**Corning New York 14831 (US)**

(72) Inventor: **Adair, Peter J.**
**131 Sewall Avenue**
**Brookline Massachusetts 02146 (US)**

(74) Representative: **Smith, Sydney et al**
**Elkington and Fife High Holborn House 52/54**
**High Holborn**
**London WC1V 6SH (GB)**

Courier Press, Leamington Spa, England.

## Description

This invention relates to dental constructs and tools and to the production thereof; more particularly, it relates to the fabrication of precision dental models, dental tools, dental appliances, dental attachments and dental prosthetic devices using mica compositions.

A major purpose of the dental profession is to replace or correct damaged or deformed tooth structure or condition by fabricating and installing dental constructs, such as dental appliances, e.g. artificial denture plates, bridges and orthodontic brackets, attachments therefor and prosthetic devices, e.g. inlays, onlays, partial or full dentures, crown and caps. Ideally, all such products: (1) should be inert in the oral environment; (2) should resist the forces of mastication; (3) should be capable of assuming physiologically compatible anatomical configuration; and (4) should exhibit aesthetic qualities similar to those of natural teeth. Dental tools are not required to meet the last three criteria, but must exhibit good strength, as well as inertness to oral environments.

Present dental constructs are customarily composed of metal alloys, porcelain, amalgam or acrylic polymers and combinations thereof, which do not completely meet the foregoing requirements. Metal alloys and amalgam are undesirable in locations where aesthetic considerations are important because they differ sharply from teeth in optical characteristics. Porcelain and acrylic polymers are either too brittle or too weak to resist masticatory forces in many locations. Composite structures, as in the case of an alloy substructure for strength and a porcelain superstructure for appearance, are extremely technique-sensitive and are too bulky in many situations. In other words, prior dental constructs have been at best a compromise among the four requirements.

DE—B—2,347,591 discloses the use in compositions for filling teeth of colourless transparent glass ceramics of the system $SiO_2$—$Al_2O_3$—$P_2O_5$—$Li_2O$—$ZrO_2$. This glass ceramic is used in the form of a filler for polymerisable filling compositions such as those containing acrylic compounds.

In US—A—3732087 there is disclosed a process for the production of tetrasilicic fluorine mica glass-ceramic articles from the controlled heat treatment of crystallizable glasses containing, in weight percent on the oxide basis, 45—70% $SiO_2$, 8—20% $MgO$, 8—15% $MgF_2$, a total of 5—35% [$R_2O$+$RO$], wherein $R_2O$ ranges from about 5—25% and consists of one or more oxides selected in the indicated proportions from the group consisting of 0—20% $K_2O$, 0—23% $Rb_2O$ and 0—25% $Cs_2O$, and wherein RO ranges from 0—20% and consists of one or more oxides selected from the group consisting of SrO, BaO and CdO, a total of 0—10% of oxides selected from the group consisting of $As_2O_5$ and $Sb_2O_5$ and up to about 5% of glass colorants. These tetrasilicic mica products exhibit good machinability with steel tools, good mechanical strength, moderate thermal expansion and good chemical durability. The use of the optional constituents $As_2O_5$, $Sb_2O_5$ and the glass colorants permits the production of translucent glass-ceramics and glass-ceramics having the appearance of marble.

The present invention is based upon the discovery that dental constructs exhibiting visual appearances similar to those of tooth enamel, having expansion coefficients and thermal conductivities approximating those of tooth enamel and demonstrating mechanical strengths at least as great as those of composite tooth structures may be readily fabricated in monolithic form from certain glass-ceramic materials as disclosed in US—A—3732087 which is discussed further below. Such materials are also useful in the fabrication of monolithic dental tools.

An early disclosure of glass-ceramics may be found in United States Patent No. 2,920,971. The production thereof comprehends three fundamental steps. Firstly, a glass-forming batch of a predetermined composition is melted. Secondly, the melt is simultaneously cooled to a temperature at least below the transformation range thereof and shaped into a glass body of a desired configuration. Thirdly, the glass shape is subjected to a particular heat treatment to cause the *in situ* development and growth of crystals therewithin such that the glass is converted into a predominantly and, frequently, essentially totally crystalline article.

The "transformation range" is defined as that temperature at which a liquid melt is deemed to have been transformed into an amorphous solid, that temperature typically being considered as lying in the vicinity of the annealing point of a glass. If desired, the glass melt may be cooled to room temperature to permit visual inspection thereof for glass quality. However, in the interests of production speed and energy economy, the commercial production of glass-ceramics commonly involves cooling the initial melt to only slightly below the transformation range and then proceeding with the crystallization heat treatment. The crystallization heat treatment commonly follows a two-step practice, *viz.* the glass body is first heated to a temperature in or somewhat above the transformation range and maintained thereat for a sufficient length of time to cause the development of nuclei and to initiate crystallization and, thereafter, the nucleated body is heated to a higher temperature, which may approach or, most often, will exceed the softening point of the glass and is held at that temperature for a sufficient length of time to effect the growth of crystals in the nuclei. These two steps have been termed "nucleation" and "crystallization", respectively.

Because a glass-ceramic is derived by the controlled crystallization of a glass, the various forming methods known to the glass technologist may be utilized in achieving a desired product shape. However, because of the highly crystalline microstructure inherent in glass-ceramics, the physical properties exhibited thereby will be more closely akin to those of the crystal phases present therein than to those of the parent glass body. As a corollary thereto, the physical properties demonstrated by whatever residual glass is included in a glass ceramic body will be quite distinct from those of the precursor glass, since the components of the crystal phase will have been removed therefrom. Furthermore, inasmuch as the glass-ceramic body results from the *in situ* crystallization of a glass, it will exhibit the same geometry as the parent glass body and be free from voids and be non-porous.

The present invention provides a monolithic dental construct or tool of a glass ceramic which has tetrasilicic fluormica as the predominant crystal phase, and which comprises in weight percent on the oxide basis, from 50.1 to 64.2% $SiO_2$, from 11.2 to 16.4% MgO, from 8.6 to 10.6% $MgF_2$, from 5 to 35% $[R_2O+RO]$ wherein $R_2O$ is from 5 to 25% and consists of at least one oxide in the indicated proportions from 0 to 15.8% $K_2O$ from 0 to 13.9% $Rb_2O$ and from 0 to 19.5% $Cs_2O$, and wherein RO is from 0 to 20% and consists of at least one oxide selected from SrO, BaO and CdO and from 0 to 2.0% $Sb_2O_3$.

The present invention further provides a process for the production of a monolithic dental construct or tool characterised in that it comprises:

(a) melting a batch for a glass-ceramic as defined above and optionally cooling to give a preform which is then remelted;

(b) shaping the melt to form a glass body having an intermediate configuration having at least one selected surface of particular conformation;

(c) heat-treating the glass body to from 650 to 850°C to develop nuclei therein and then heat treating the body at from 800 to 1200°C to grow tetrasilicic fluormica crystals on the nuclei and thereby convert it into a glass-ceramic body of corresponding intermediate shape wherein tetrasilicic fluormica constitutes the predominant crystal phase; and

(d) forming selected surfaces of the glass-ceramic body as desired to provide the monolithic dental construct or tool.

More particularly, such a process may involve:

(a) pressing a soft impression material against a dental surface to establish a shape and solidifying the shape to form an impression;

(b) filling the impression with dental stone material and solidifying the dental stone material to form a model;

(c) pressing a soft impression material against the model to define conforming surfaces and shaping the remainder of the surfaces thereof to form a pattern;

(d) placing the pattern in an investment casting slurry on a sprue that extends from the pattern to a surface of the slurry and solidifying the slurry to form a mould;

(e) removing the sprue and the pattern from the mould;

(f) melting a glass ceramic preform having a composition as defined above;

(g) heating the mould to an elevated temperature, but below the melting temperature of the glass preform;

(h) casting the melt into the mould to form a glass body therein;

(i) removing the glass body from the mould;

(j) heat treating the glass body as defined above; and

(k) forming the glass-ceramic body as desired to provide a monolithic dental construct or tool.

In general terms, the process according to the present invention contemplates four basic elements. Firstly, a glass-forming batch of a desired composition is melted. Secondly, the melt is shaped, for example by casting into a mould, compression moulding, centrifugal casting or injection moulding, to form a glass body having an intermediate configuration having at least one selected surface of particular conformation. Thirdly, the glass body is heat treated in a particular manner to crystallize it *in situ* and thereby convert it into a glass-ceramic body of corresponding intermediate shape. Fourthly, selected surfaces of the glass-ceramic body will be formed, for example machined, into a dental tool or construct of final shape.

As indicated above, the glass-ceramic compositions operable as dental constructs are limited by the constraints that they:

(a) be inert in the oral environment;

(b) be sufficiently strong to resist the forces of mastication, i.e. exhibit a tensile strength as defined in terms of modulus of rupture greater than 560 Kg/sq.cm (8000 psi);

(c) be capable of assuming physiologically compatible anatomical configuration;

(d) have coefficients of thermal expansion and thermal conductivities approximating those of tooth enamel; and

(e) will preferably exhibit a visual appearance similar to that of tooth enamel. The last-mentioned requirement is not absolutely mandatory since an outer layer, e.g. porcelain, may be applied thereto. However, such practice adds expense and involves careful matching of the properties of the porcelain and substrate material. The material for dental tools must be inert in the oral environment, possess a modulus of rupture greater than 560 Kg/sq.cm. (8000 psi) and be capable of ready shaping.

As mentioned above, United States Patent No. 2,920,971 may be considered as the basic disclosure in the field of glass-ceramics and numerous exemplary compositions are set forth therein. However, as has been alluded to above, the final configuration of dental constructs and dental tools is customarily achieved by machining of the body material. This capability of being mechanically shaped, in particular machined, with relative ease utilizing conventional steel tools is particularly demonstrated in glass-ceramics wherein a mica constitutes the predominant crystal phase. Numerous glass-ceramics containing synthetic fluormica crystals are known.

Mica-containing glass-ceramics demonstrate a relatively unique property which renders them particularly desirable in applications, such as dental tools and constructs. Thus, such bodies manifest deviations from brittle behaviour which permit them to withstand point impact without fracture propagation. For example, these bodies may be indented in a point hardness test procedure where conventional porcelains are fractured. This capability of mica-containing glass-ceramics is due to the fact that the crystal phase may flow plastically to some extent by translational gliding along the basal or cleavage plane.

United States Patent No. 3,689,293 discloses glass-ceramic bodies demonstrating excellent machinability accompanied by good mechanical strength and impact resistance. These glass-ceramics contain fluorophlogopite solid solution as the predominant crystal phase and have an overall composition consisting essentially, by weight on the oxide basis, of from 25 to 60% $SiO_2$, from 15 to 35% $R_2O_3$, wherein $R_2O_3$ consists of from 3 to 15% $B_2O_3$ and from 5 to 25% $Al_2O_3$, from 2 to 20% $R_2O$, wherein $R_2O$ consists of from 0 to 15% $Na_2O$, from 0 to 15% $K_2O$, from 0 to 15% $Rb_2O$ and from 0 to 20% $Cs_2O$, from 6 to 25% $MgO+Li_2O$ consisting of from 4 to 25% $MgO$ and from 0 to 7% $Li_2O$ and from 4 to 20% F. The precursor glass bodies are converted to glass-ceramics by heat treatment at temperatures of from 750 to 1100°C. The preferred heat treatment consists of nucleating at from 750 to 850°C., followed by crystallization at from 850 to 1100°C. Such products may be very readily shaped into dental constructs and dental tools.

However, although not as readily machinable as the materials according to U.S. Patent No. 3,689,293, the most preferred compositions for use as dental constructs and dental tools are those disclosed in United States Patent No. 3,732,087. These latter compositions not only demonstrate somewhat superior chemical durability and mechanical strength, e.g. modulus of rupture values up to 2100 Kg/sq.cm. (30,000 psi), but also exhibit two other very important features, one cosmetic and the other of practical significance. Firstly, the crystallized products closely approximate the translucency-opacity characteristic of the appearance of natural teeth. Secondly, the materials display wearing properties quite similar to those of natural teeth, i.e. the hardness and abrasion resistance are closely comparable such that the glass-ceramic product wears at about the same rate as natural teeth. This latter faculty makes for long term comfort and efficient mastication.

The glass-ceramic materials according to U.S. Patent No. 3,732,087 demonstrate good machinability and contain tetrasilicic mica as the predominant crystal phase. The base compositions therefor consist essentially, by weight on the oxide basis as calculated from the batch, of from 45 to 70% $SiO_2$, from 8 to 20% $MgO$, from 8 to 15% $MgF_2$, from 5 to 35% $R_2O+RO$, wherein $R_2O$ is from 5 to 25% and consists of at least one oxide selected in the indicated proportion from: from 0 to 20% $K_2O$, from 0 to 23% $Rb_2O$ and from 0 to 25% $Cs_2O$, and wherein RO is from 0 to 20% and consists of at least one oxide selected from SrO, BaO and CdO. As optional ingredients, up to 10% $Sb_2O_5$ and/or up to 5% of one or more conventional glass colourants may be present. The parent glass bodies are crystallised *in situ* to glass-ceramics by nucleating at from 650 to 850°C., followed by crystallization at from 800 to 1200°C. As observed therein, a period of from 0.25 to 10 hours is generally sufficient to induce nucleation and from 1 to 100 hours will customarily be utilized in the crystallization step to ensure a high proportion of crystals in the product. Compositions consisting essentially of from 55 to 65% $SiO_2$, from 12 to 20% $MgO$, from 9 to 13% $MgF_2$, from 7 to 18% $K_2O$ and from 0.5 to 8% $As_2O_5$ are preferred for the machinability thereof.

Referring to the accompanying drawings:

Figure 1 illustrates producing an investment mould in accordance with the present invention;

Figure 2 illustrates forming a parent glass casting in accordance with the present invention;

Figure 3 illustrates heat treating the casting to form a glass-ceramic component in accordance with the present invention; and

Figure 4 illustrates machining the glass-ceramic component to provide a final dental construct.

Firstly, an impression 10 is formed conventionally by pressing a soft dental impression composition, e.g. silicon rubber, wax or mercaptan rubber, against prepared dental surfaces of specified configuration and solidifying the resulting shape. Then, this shape is filled with dental stone, e.g. plaster of Paris, to form a master model 12. A pattern 20 in, say, wax of the dental construct, characterized by a sprue 14 and a pattern body 16, is prepared in association with the master model. In this case, the prepared dental surfaces are presented by the prepared facets of a tooth to be restored and the specified configuration is shown as involving the reentrant, but not undercut, inward facets 21 of a restoration having an anatomical outward surface 22. Then, pattern 20 is imbedded in a refractory investment slurry 24, which is permitted to solidify. The investment is heated to remove the wax, for example, from the resulting mould cavity 26. The investment slurry material is typically a phosphate-bonded or silicate-bonded inert cementitious clay. A batch of a predetermined composition is heated to a temperature of from 1325 to 1500°C. in a refractory crucible composed, for example, of platinum, alumina, silica, mullite or zirconia. The resultant melt is injected into the mould, which normally will have been heated to a temperature of from 700 to 950°C. to forestall cracking or breakage thereof from thermal shock, under a back air pressure of, for example, from 1.6 to 4.5 Kg/sq.cm. (from 8 to 50 psig). A vacuum may be applied in conjunction with the back air pressure to assist in ensuring complete filling or, if desired, sufficient vacuum may be applied alone to suck the melt into the mould. In general, the vacuum will range from 0.2 to 1.0 bar (1 bar=10⁶ dynes/sq.cm.). Also, as may be appreciated, mechanical means, such as a piston, injection moulding or centrifugal casting may be utilized to fill the mould cavity. Centrifugal forces of from 1.1 to 2.0 Kg/sq.cm. (from 1 to 15 psig) have been found satisfactory for this purpose.

Under these circumstances, the contraction rate of the mould cavity during cooling will closely match the contraction rate of the melt so that little or no compression is exerted by the mould on the casting. Initially, the elevated temperature of the melt does not affect the temperature of the mould because the mass of the melt is relatively small. Then, the mould and its contents are allowed to cool to room temperature and a clear parent glass casting 28, certain of its surfaces 30 being of the original specified configuration, is removed from the mould. The transparency permits the casting to be readily inspected visually for flaws. As shown, the parent glass casting 28 generally is in the shape of a cap having in addition to dome-shaped surface 32, a residual sprue 34 and button 35, which have resulted from the afore-mentioned casting steps. Then parent glass casting 28, touching only sprue 34, is heat treated at a temperature and for a time sufficient to cause *in situ* crystallization thereof such that the casting is converted from a glass to a predominantly crystalline body. Then, certain surfaces of this dental component are machined, employing conventional dental drills and mills, to produce the desired shape. As shown, glass-ceramic component 36 is ground at 38 to sever sprue 34 and to provide a polished, anatomically shaped surface. The outer surface of the component optionally thereafter is polished to provide a smooth and glossy appearance. Also, if desired, the dental component is optionally coloured and/or glazed to conform the appearance of the component to that of tooth structure with which it is to be associated.

In the tetrasilicic fluorine micas which crystallize from the starting glasses to form the preferred glass ceramic materials, the X, Y and Z positions are believed to be filled in the following manner: X position K, Rb, Cs, Sr, Ba or Cd as available; Y position Mg only; and Z position Si only. These micas, which normally have the postulated formula: $KMg_{2.5}Si_4O_{10}F_2$, are described as tetrasilicic because they do not display Al- or B-for-Si substitutions in the $Z_2O_5$ hexagonal sheets of the mica layer as do the fluorophlogopites $(KMg_3AlSi_3O_{10}F_2)$ or $(KMg_3BSi_3O_{10}F_2)$, such as comprise the predominant crystal phases in the products according to U.S. Patent No. 3,689,293. Those crystals have been termed trisilicic fluormicas. In contrast, it appears that tetrasilicic fluormica is the primary crystalline species present in the preferred glass-ceramics, the common variations being in which of the $K^+$, $Rb^+$, $Cs^+$, $Sr^{2+}$, $Ba^{2+}$ or $Cd^{2+}$ ions (or combinations thereof) occupy the X or interlayer positions. The Y positions are believed to be almost exclusively occupied by $Mg^{2+}$ ions and very few deficiencies in either the X or Y positions are expected. Thus, the presently preferred glass-ceramics are related to prior art mica glass-ceramics, e.g. those according to U.S. Patent No. 3,689,293, in that they contain synthetic mica crystals, but they are distinguishable on the basis that they do not contain trivalent cations, such as $Al^{3+}$ and $B^{3+}$, as essential crystal constituents. Minor additions of other oxides, such as $P_2O_5$, $TiO_2$, $ZrO_2$, FeO, ZnO, $GeO_2$, MnO, $La_2O_3$ and $SnO_2$, to the base glass composition may be tolerated to a total of 10%, by weight, and are useful, for example, in controlling the properties of the parent glass and the residual glassy phase. It will be appreciated that, if desired, a minor amount of $Al_2O_3$ and/or $B_2O_3$ may be included in the base composition which may alter the growth habits of the crystal phase.

In general, the dental laboratory will not melt the batch materials to produce the precursor glass since very high temperatures and stirring are utilized to ensure a homogeneous body. Rather, the dental laboratory will commonly purchase the precursor glass from a glass manufacturer in some convenient form, e.g. buttons or marbles. This glass preform may then be remelted in the laboratory and will be poured into a mould at a temperature above its liquidus or otherwise shaped to form a glass body having at least one surface of a particular conformation. Heat treatment is effected after the melt has been cooled below its transformation range and is continued until nuclei are first formed throughout the glass, followed by the growth of fluormica crystals on those nuclei.

The resulting glass-ceramic compositions are such that they are typically characterized by a white or off-white colour, unless colourants have been deliberately added to the batch. The intermediate glass component has a characteristic clear or somewhat hazy vitreous structure. The final glass-ceramic product consists essentially of mica crystals homogeneously dispersed within a residual glassy matrix, the crystals constituting the predominant proportion of the body. In general, the higher the proportion of crystals, the more desirable the product.

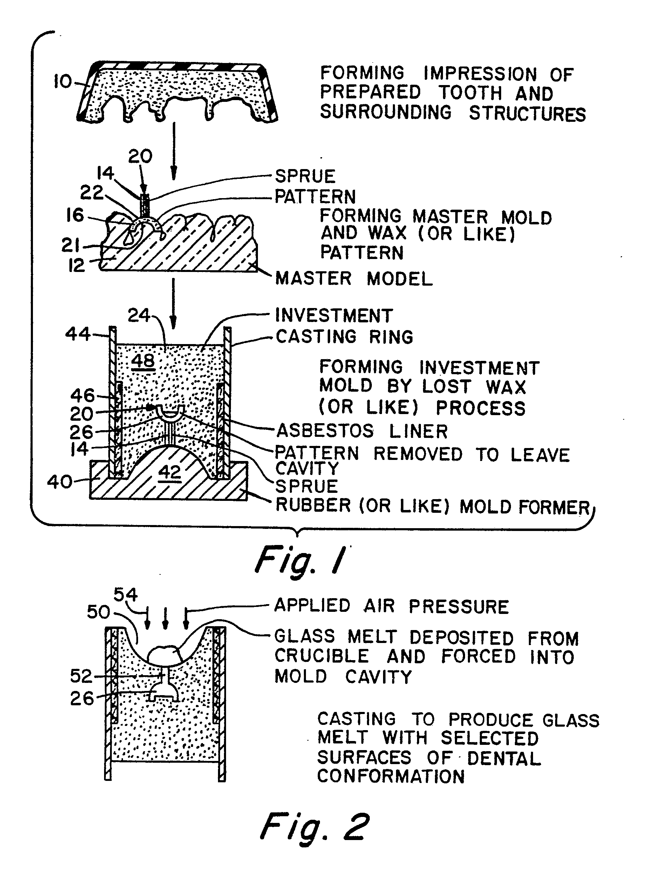

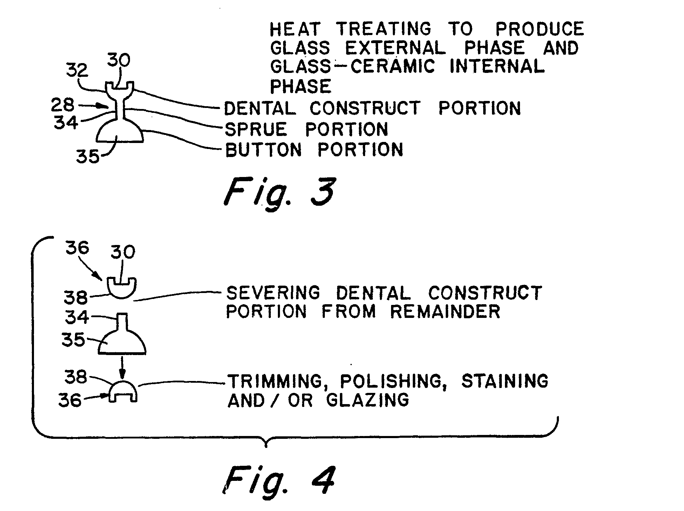

The present process contemplates the production of a variety of dental tools and constructs of the abovementioned compositions. The dental constructs considered herein are deemed to fall under the four general categories of dental models, dental appliances, dental attachments and prosthetic devices. Typically, the inlays are of the type that have inward walls or facets that conform to prepared internal walls or facets of a tooth and outward walls or facets that are in continuity with the external contour of the tooth. Customarily, as described above with reference to accompanying Figures 1 to 4, the caps or crowns are of the type that fit over and cover the prepared crown form or root canal post of a tooth stump, having lower inward walls or facets that conform to prepared outer walls or facets of the tooth stump and upper outward walls or facets that are in continuity with the external contour of the tooth. Commonly, the prostheses are of the type that replace dental and/or related structures in the oral cavity, for example, false teeth, dentures and components thereof.

5

**0 022 655**

Example

With reference to the accompanying drawings, the process according to the present invention comprises the following illustrative steps for producing a dental restoration. A wax pattern is formed conventionally and, as shown at 20, is mounted on the upper end of sprue 14. The lower end of the sprue is attached to a cylindrical casting form 40 by a soft wax bond 42. Wax bond 42 is manipulated to provide continously smooth joints. Wax pattern 20 is painted with polar surfactant solution, e.g. either aqueous or alcoholic, to minimize tackiness and is blown dry using an air stream. Wax pattern 20 is surrounded by a metal casting ring 44, which has an asbestos liner 46. An investment slurry 48 is prepared by mixing a refractory, such as silica flour, and an aqueous liquid, such as water/ethyl silicate solution. The investment slurry is first painted onto the wax pattern and then is poured into the casting ring so as to cover the pattern completely, but to a height of no more than about 1.25 centimetres above the wax pattern. The investment slurry is allowed to set for approximately 45 minutes to form a green investment mould. To cure this green investment mould, it is placed into a cold furnace, heated to approximately 650°C. in a 1 hour period of gradually increasing temperature and is maintained at approximately 650°C. for 1 hour period of steady temperature.

The curved investment mould is thereafter removed from the casting form and casting ring and inverted to provide precision cavity 26 which communicates with a dished upper mouth 50 though a port 52, the wax and the plasic tube having been burned out during the curing period. Into this cavity through a suitable port is poured the melt of, for example, a tetrasilicic fluormica composition, which has been heated in a suitable crucible to a temperature providing adequate fluidity. The melt is forced through depression 50 and port 52 into cavity 26 by a backup air pressure 54 of approximately 0.56 Kg/sq.cm. (8 psi), which is maintained until the melt has solidified to a glass.

After the casting is cooled to room temperature, the bulk of the investment material is removed mechanically from the glass casting and residual adhering fragments are removed by application of an investment solvent liquid and by ultrasonic energy. Then, the parent glass casting (after visual inspection for possible casting flaws) is mounted by button 34 and unsupported other than by the sprue and button, in a furnace. The temperature within the furnace is raised slowly at about 200°C./hour to from 1050 to 1150°C., maintained thereat for about 4 hours and thereafter cooled. Then, sprue 34 and button 34 are removed by grinding and the surfaces of the cap are ground to finally adjusted shape.

It will be appreciated that, if desired, the parent glass casting may be heat treated while within the investment mould to effect crystallization thereof. This practice has the advantages of speeding production and fuel economy. Thus, rather than cooling the glass to room temperature and then reheating, the glass need only be cooled to below the transformation range thereof and thereafter reheated to the nucleation and crystallization temperature ranges. The investment material will then be removed mechanically from the crystallized casting. However, it is apparent that this practice does not permit inspection of the casting for flaws in the glass casting prior to crystallization.

Specific exemplary formulations of the glass starting materials are given in Table I below.

TABLE I

Weight percent

|  | 1 | 2 | 3 | 4 | 5 | 6 | 7 |
|---|---|---|---|---|---|---|---|
| $K_2O$ | 13.8 | 15.8 | 9.0 | 11.0 | 7.5 | — | — |
| $Rb_2O$ | — | — | — | — | — | 13.9 | — |
| $Cs_2O$ | — | — | — | — | — | — | 19.5 |
| BaO | — | — | — | — | 12.2 | 11.4 | 10.6 |
| SrO | — | — | — | — | — | — | — |
| $MgF_2$ | 10.6 | 10.4 | 10.6 | 10.6 | 9.9 | 9.3 | 8.6 |
| MgO | 13.8 | 13.5 | 16.2 | 16.4 | 12.9 | 12.0 | 11.2 |
| $SiO_2$ | 61.8 | 60.3 | 64.2 | 62.0 | 57.5 | 53.4 | 50.1 |
| $Sb_2O_3$ | — | — | — | — | — | — | — |

Specific exemplary heat treating times and temperatures for crystallizing the glasses reported in Table I into glass-ceramics are given in Table II below:

6

**0 022 655**

TABLE II

| Example No. | | | |
|---|---|---|---|
| 1 | 800°C. for 4 hours, | 1090°C. for 4 hours | Fine grain fracture, off white, slightly translucent |
| 2 | 800°C. for 4 hours, | 1090°C. for 4 hours | Fine cherty fracture, white, slightly translucent |
| 3 | 800°C. for 4 hours, | 1090°C. for 4 hours | Fine cherty fracture, white, opaque |
| 4 | 800°C. for 4 hours, | 1150°C. for 4 hours | Fine grain fracture, white, opaque |
| 5 | 800°C. for 4 hours, | 1150°C. for 4 hours | Coarse sugary fracture, slightly translucent |
| 6 | 800°C. for 4 hours, | 1125°C. for 4 hours | Medium grain sandy fracture, white opaque |
| 7 | 800°C. for 4 hours, | 1125°C. for 4 hours | Medium grain cherty fracture, white, slightly translucent |

The white or off-white, translucent-opaque colour common to those crystalline materials is modified in various embodiments by, for example, the addition of known colouring agents, such an manganese oxide, which produces a pinkish hue, iron, which produces a yellowish hue, and nickel oxide, which produces a grayish hue, or by the addition of modifying agents, such as cerium oxide, titanium oxide and zirconium oxide.

The characteristics of the glass-ceramic product of Example 2 above are as follows:

| | |
|---|---|
| Ultimate tensile strength | 1828 Kg/sq.cm. (26,000 psi) |
| Compressive strength | 5273 Kg/sq.cm. (75,000 psi) |
| Modulus of elasticity | $9.86 \times 10^6$ |
| Shear modulus | $3.94 \times 10^6$ |
| Poisson's ratio | 0.25 |
| Coefficient of linear expansion | $83.3 \times 10^{-7}$/°C. |
| Density | 2.61 g/cc |
| Porosity | 0 |
| Thermal conductivity of 25°C. | 0.003 cal cm/sec cm²°C. |
| Machinability | similar to metals |
| X-ray translucency | similar to dentine |
| Tissue tolerance | good |
| Cementation bond strength | good |

**Claims**

1. A monolithic dental construct or tool of a glass ceramic which has tetrasilicic fluormicà as the predominant crystal phase, and which comprises in weight percent on the oxide basis, from 50.1 to 64.2% $SiO_2$, from 11.2 to 16.4% MgO, from 8.6 to 10.6% $MgF_2$, from 5 to 35% [$R_2O+RO$] wherein $R_2O$ is from 5 to 25% and consists of at least one oxide in the indicated proportions from: from 0 to 15.8% $K_2O$ from 0 to

7

13.9% $Rb_2O$ and from 0 to 19.5% $Cs_2O$, and wherein RO is from 0 to 20% and consists of at least one oxide selected from SrO, BaO and CdO and from 0 to 2.0% $Sb_2O_3$.

2. A process for the production of a dental construct or tool according to claim 1, characterised in that it comprises:

(a) melting a batch for a glass-ceramic as defined in claim 1 and optionally cooling to give a preform which is then remelted;

(b) shaping the melt to form a glass body having an intermediate configuration having at least one selected surface of particular conformation;

(c) heat treating the glass body to from 650 to 850°C to develop nuclei therein and then heat treating the body at from 800 to 1200°C to grow tetrasilicic fluormica crystals on the nuclei and thereby convert it into a glass-ceramic body of corresponding intermediate shape wherein tetrasilicic fluormica constitutes the predominant crystal phase; and

(d) forming selected surfaces of the glass-ceramic body as desired to provide a monolithic dental construct or tool.

3. A process according to claim 2 comprising:

(a) pressing a soft impression material against a dental surface to establish a shape and solidifying the shape to form an impression 10;

(b) filling the impression with dental stone material and solidifying the dental stone material to form a model 12;

(c) pressing a soft impression material against the model to define conforming surfaces and shaping the remainder of the surfaces thereof to form a pattern 20;

(d) placing the pattern in an investment casting slurry 24 on a sprue 14 that extends from the pattern to a surface of the slurry and solidifying the slurry to form a mould;

(e) removing the sprue and the pattern from the mould 36;

(f) melting a glass cermaic preform having a composition as defined in claim 1;

(g) heating the mould to an elevated temperature, but below the melting temperature of the glass preform;

(h) casting the melt into the mould to form a glass body therein;

(i) removing the glass body from the mould;

(j) heat treating the glass body as defined in claim 2; and

(k) forming the glass-ceramic body as desired to provide a monolithic dental construct or tool.

4. A process according to claim 2 or claim 3 wherein the batch also comprises up to 10% $As_2O_5$ and/or up to 5% of one or more glass colourants.

5. A process according to any of claims 2 to 4 wherein the period to develop nuclei is from 0.25 to 10 hours and the period to grow tetrasilicic fluormica crystals is from 1 to 100 hours.

6. A process according to any of claims 2 to 5 wherein the glass body is heat treated in a substantially unsupported condition.

7. A process according to any of claims 2 to 6 wherein the glass preform is melted at from 1325 to 1500°C.

8. A process according to any of claims 3 to 7 wherein the mould is heated to from 700 to 950°C.

9. A process according to any of claims 3 to 8 wherein the melt is introduced into the mould by injection under a back air pressure or by suction using a vacuum or by centrifugal force.

10. A process according to claim 9 wherein a back air pressure of from 1.6 to 4.5 Kg/sq.cm. is used or a vacuum of from 0.2 to 1 bar is used or a centrifugal force of from 1.1 to 2.0 Kg/sq.cm. is used.

**Patentansprüche**

1. Monolithischer Dental-Aufbau oder monolithisches Dental-Werkzeug aus Glaskeramik mit Tetrasilicium-Fluorglimmer als vorherrschende Kristallphase, bestehend in Gewichtsprozent auf Oxidbasis aus 50,1 bis 64,2 % $SiO_2$, 11,2 bis 16,4 % MgO, 8,6 bis 10,6 % $MgF_2$, 5 bis 35 % [$R_2O$+RO] wobei $R_2O$ von 5 bis 25 % enthalten ist und wenigstens aus einem Oxid der angegebenen Verhältnisse besteht: 0 bis 15,8 % $K_2O$, 0 bis 13,9 % $Rb_2O$ und 0 bis 19,5 % $Cs_2O$, und wobei RO von 0 bis 20 % enthalten ist und wenigstens aus einem aus SrO, BaO und CdO ausgewählten Oxid und aus 0 bis 2,0 % $Sb_2O_3$ besteht.

2. Verfahren zur Herstellung eines Dental-Aufbaus oder eines Dental-Werkzeugs nach Anspruch 1, gekennzeichnet durch die folgenden Verfahrensschritte:

a) Schmelzen eines Ansatzes für eine Glaskeramik gemäß Anspruch 1 und wahlweises Abkühlen zu einem Rohling, der dann wieder geschmolzen wird;

b) Formen der Schmelze zur Bildung eines Glaskörpers mit einer Zwischenkonfiguration, die wenigstens eine ausgewählte Oberfläche einer besonderen Gestaltung aufweist;

c) Wärmebehandlung des Glaskörpers bei einer Temperatur von 650 bis 850°C, um in ihm Kristallisationskerne zu entwickeln, und anschließende Wärmebehandlung des Körpers bei einer Temperatur von 800 bis 1200°C, um Tetrasilicium—Fluorglimmerkristalle (tetrasilicic fluormica crystals) an den Kristallisationskernen waschen zu lassen und ihn dadurch in einen glaskeramischen Körper einer entsprechenden Zwischenform umzuwandeln, bei der Tetrasilicium-Fluorglimmer die vorherrschende Kristallphase bildet; und

d) Formen ausgewählter Oberflächen des glaskeramischen Körpers nach Bedarf, um einen monolithischen Dental-Aufbau bzw. ein monolithisches Dental-Werkzeug zu schaffen.

3. Verfahren nach Anspruch 2, gekennzeichnet durch

a) Drücken eines weichen Abdruckmaterials gegen eine Zahnoberfläche zur Gewinnung einer Form und Aushärtenlassen der Form zur Bildung eines Abdrucks (10);

b) Füllen des Abdrucks mit dentalem Steinmaterial und Aushärtenlassen des dentalen Steinmaterials zur Bildung eines Modells (12);

c) Drücken eines weichen Abdruckmaterials gegen das Modell zur Bildung angepaßter Oberflächen und Formen deren restlicher Oberflächen zur Bildung eines Modells (20);

d) Anordnen des Modells in einen Hüllgußschlamm (24) auf einem Anguß (14), der sich von dem Muster zu einer Oberfläche des Schlamms erstreckt, und Aushärtenlassen des Schlamms zur Bildung einer Form;

e) Entfernen des Angusses und des Musters aus der Form (36);

f) Schmelzen eines glaskeramischen Rohlings mit einer Zusammensetzung gemäß Anspruch 1;

g) Erhitzen der Form auf eine erhöhte Temperatur, jedoch unterhalb der Schmeltztemperatur des Glasrohlings;

h) Gießen der Schmelze in die Form, um darin einen Glaskörper zu bilden;

i) Entfernen des Glaskörpers aus der Form;

j) Wärmebehandlung des Glaskörpers gemäß Anspruch 2; und

k) Gestalten des glaskeramischen Körpers nach Bedarf zur Schaffung eines monolithischen Dental-Aufbaues bzw. monolithischen Dental-Werkzeugs.

4. Verfahren nach Anspruch 2 oder 3, dadurch gekennzeichnet, daß der Ansatz auch bis zu 10 % $As_2O_5$ und/oder bis zu 5 % eines oder mehrerer Glasfärbungsmittel enthält.

5. Verfahren nach einem der Ansprüche 2 bis 4, dadurch gekennzeichnet, daß der Zeitraum zur Entwicklung der Kristallisationskerne von 0,25 bis 10 Stunden beträgt, und daß der Zeitraum für das Wachsenlassen der Tetrasilicium-Fluorglimmer-Kristalle von 1 bis 100 Stunden beträgt.

6. Verfahren nach einem der Ansprüche 2 bis 5, dadurch gekennzeichnet, daß der Glaskörper in einem im wesentlichen nicht gestützen Zustand warm behandelt wird.

7. Verfahren nach einem der Ansprüche 2 bis 6, dadurch gekennzeichnet, daß der Glasrohling bei einer Temperatur von 1325 bis 1500°C geschmolzen wird.

8. Verfahren nach einem der Ansprüche 3 bis 7, dadurch gekennzeichnet, daß die Form auf 700 bis 950°C erhitzt wird.

9. Verfahren nach einem der Ansprüche 3 bis 8, dadurch gekennzeichnet, daß die Schmelze in die Form durch Einspritzen unter einem Zusatzluftdruck oder durch Einsaugen unter Verwendung eines Unterdrucks oder durch Zentrifugalkraft eingeführt wird.

10. Verfahren nach Anspruch 9, dadurch gekennzeichnet daß ein Zusatzluftdruck von 1,6 bis 4,5 kg/cm$^2$ oder ein Unterdruck von 0,2 bis 1 bar oder eine Zentrifugalkraft von 1,1 bis 2,0 kg/cm$^2$ verwendet werden.

**Revendications**

1. Une construction ou outil dentaire monolitique en une vitrocéramique qui a du fluoromica tétrasilicique comme phase cristalline prédominante, et qui comprend en % en poids sur la base des oxydes, de 50,1 à 64,2 % de $SiO_2$, de 11,2 à 16,4 % de MgO, de 8,6 à 10,6 % de $MgF_2$, de 5 à 35 % de $[R_2O+RO]$ où $R_2O$ représente de 5 à 25 % et consiste en ou moins un oxyde dans les proportions suivantes: de 0 à 15,8 % de $K_2O$, de 0 à 13,9 % de $Rb_2O$ et de 0 à 19,5 % de $Cs_2O$, et où RO représente de 0 à 20 % et consiste en au moins un oxyde choisi parmi SrO, BaO et CdO, et de 0 à 2,0 % de $Sb_2O_3$.

2. Un procédé pour la production d'une construction ou outil dentaire selon la revendication 1, caractérisé en ce qu'il comprend:

(a) la fusion d'une charge de départ pour une vitrocéramique telle que définie à la revendication 1 et son refroidissement facultatif pour obtenir une préforme qui est ensuite refondue;

(b) la mise de la masse fondue à la forme d'un corps en verre ayant une configuration intermédiaire ayant au moins une surface choisie de conformation particulière;

(c) le traitement thermique du corps en verre à une température de 650 à 850°C pour y développer des germes, puis le traitement thermique du corps à une température de 800 à 1200°C pour faire croître des cristaux de fluoromica tétrasilicique sur les germes et le convertir ainsi en un corps de vitrocéramique de forme intermédiaire correspondant dans lequel le fluoromica tétrasilicique constitue la phase cristalline prédominante; et

(d) le formage de surfaces choisies du corps de vitrocéramique comme désiré pour obtenir une construction ou outil dentaire monolithique.

3. Un procédé selon la revendication 2, comprenant:

(a) le pressage d'une matière molle pour empreintes contre une surface dentaire pour établir une forme et la solidification de la forme pour former une empreinte 10;

(b) le remplissage de l'empreinte avec de la matière du type pierre dentaire pour former un modèle 12;

(c) le pressage d'une matière molle pour empreintes contre le modèle pour définir des surfaces épousant la forme du modèle et la conformation du reste des surfaces pour former un gabarit 20;

(d) la mise en place du gabarit dans une bouillie 24 de moulage à la cire perdue sur une baguette de coulèe 14 qui s'étend du gabarit jusqu'à une surface de la bouillie, et la solidification de la bouillie pour former un moule;

(e) l'élimination de la baguette de coulée et du gabarit à partir du moule 36;

(f) la fusion d'une préforme de vitrocéramique ayant une composition telle que définie à la revendication 1;

(g) le chauffage du moule à une température élevée mais inférieure à la température de fusion de la préforme de verre;

(h) la coulée de la masse fondue dans le moule pour y former un corps de verre;

(i) l'enlèvement du corps de verre à partir du mould;

(j) un traitement du corps de verre comme défini dans la revendication 2; et

(k) un formage du corps de vitro-céramique comme désiré pour obtenir une construction ou outil dentaire monolithique.

4. Un procédé selon la revendication 2 ou 3, dans lequel la charge de départ comprend aussi jusqu'à 10 % d'$As_2O_5$ et/ou jusqu'à 5 % d'un ou plusieurs colorants du verre.

5. Un procédé selon l'une quelconque des revendications 2 à 4, dans lequel la période de développement des germes va de 0,25 à 10 heures et la période de croissance des cristaux de fluoromica tétrasilicique va de 1 à 100 heures.

6. Un procédé selon l'une quelconque des revendications 2 à 5, dans lequel le corps de verre est traité thermiquement dans un état substantiellement non supporté.

7. Un procédé selon l'une quelconque des revendications 2 à 6, dans lequel la préforme de verre est fondue à une température de 1325 à 1500°C.

8. Un procédé selon l'une quelconque des revendications 3 à 7, dans lequel le moule est chauffé à une température de 700 à 950°C.

9. Un procédé selon l'une quelconque des revendications 3 à 8, dans lequel la masse fondue est introduite dans le moule par injection sous une pression d'air à l'arrière ou par suction en utilisant un vide ou par l'effet de la force centrifuge.

10. Un procédé selon la revendication 9, dans lequel on utilise une pression d'air à l'arrière de 1,6 à 4,5 kg/cm² ou un vide de 0,2 à 1 bar ou une force centrifuge de 1,1 à 2,0 kg/cm².

FORMING IMPRESSION OF
PREPARED TOOTH AND
SURROUNDING STRUCTURES

10

20
14 — SPRUE
22 — PATTERN
16 FORMING MASTER MOLD
AND WAX (OR LIKE)
21 PATTERN
12 — MASTER MODEL

24 — INVESTMENT
44 — CASTING RING

48 FORMING INVESTMENT
MOLD BY LOST WAX
(OR LIKE) PROCESS
46
20 — ASBESTOS LINER
26 — PATTERN REMOVED TO LEAVE
14 CAVITY
40 42 — SPRUE
RUBBER (OR LIKE) MOLD FORMER

*Fig. 1*

54 — APPLIED AIR PRESSURE
50 — GLASS MELT DEPOSITED FROM
CRUCIBLE AND FORCED INTO
MOLD CAVITY

52
26 CASTING TO PRODUCE GLASS
MELT WITH SELECTED
SURFACES OF DENTAL
CONFORMATION

*Fig. 2*

HEAT TREATING TO PRODUCE
GLASS EXTERNAL PHASE AND
GLASS-CERAMIC INTERNAL
PHASE

DENTAL CONSTRUCT PORTION
SPRUE PORTION
BUTTON PORTION

*Fig. 3*

SEVERING DENTAL CONSTRUCT
PORTION FROM REMAINDER

TRIMMING, POLISHING, STAINING
AND / OR GLAZING

*Fig. 4*